# EUROPEAN PATENT APPLICATION

(11) **EP 3 444 354 A1**
(43) Date of publication of application: **20.02.2019**
(21) Application number: 17186455.6
(22) Date of filing: 16.08.2017
(51) Int. Cl.: C12P 17/04, C07D 307/46, C12M 1/00, B01J 19/00

(54) **PROCESS OF SEPARATING 2,5-DIFORMYLFURAN FROM AN AQUEOUS MIXTURE BY COOLING**

(71) Applicant: BASF SE, 67056 Ludwigshafen am Rhein (DE)
(72) Inventor: Breuer, Michael, 67056 Ludwigshafen (DE); Baldenius, Kai-Uwe, 67056 Ludwigshafen (DE)
(74) Representative: BASF IP Association

(57) **Abstract**

Described is a process of separating 2,5-diformylfuran from an aqueous mixture comprising dissolved 5-(hydroxymethyl)furfural, dissolved 2,5-diformylfuran and a buffer substance, comprising the step of (a) cooling down the mixture so that 2,5-diformylfuran precipitates, wherein the aqueous mixture preferably additionally comprises one or more compounds selected from the group consisting of oxidoreductase, horse radish peroxidase, and catalase. Also described is a corresponding process of biocatalytic oxidation of 5-(hydroxymethyl)furfural to 2,5-diformylfuran, and a corresponding apparatus.

## Description

The present invention relates to a process of separating 2,5-diformylfuran from an aqueous mixture. The invention furthermore relates to a process of biocatalytic oxidation of 5-(hydroxymethyl)furfural to 2,5-diformylfuran, comprising a step of separating 2,5-diformylfuran from an aqueous mixture by the corresponding process of the invention. The invention also relates to an apparatus for use in a process of biocatalytic oxidation of the present invention, and to the corresponding use of an apparatus of the invention in a process of the invention.

The invention is defined in the attached claims. The present description discloses the invention and specific embodiments therof, as well as related aspects.

5-(hydroxymethyl)furfural (HMF; CAS: 67-47-0; 1) is a high value platform chemical based on renewable resources. 5-(hydroxymethyl)furfural is generally accessible from renewable raw materials, e.g. by dehydration of fructose. It is generally preferred to convert 5-(hydroxymethyl)furfural to compounds with a homogeneous redox state, in order to simplify follow-up chemistry. In particular, 2,5-diformylfuran (DFF; CAS: 823-82-5; 2) is an important oxidation product of 5-(hydroxymethyl)furfural, which can be used as a starting material for polymerization or other follow-up-conversions. More specifically, 2,5-diformylfuran can be used as a building block and cross-linking agent in a range of different applications. For example, 2,5-diformylfuran can be used as a monomer for polymer production, e.g., in combination with urea, or can be further oxidized to useful building blocks such as formylfuran carboxylic acid (FFCA) and 2,5-furan dicarboxylic acid (FDCA).

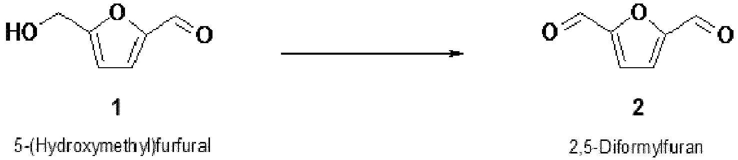

WO 2014/015256 discloses enzymatic methods for oxidation of 5-(hydroxymethyl)furfural and derivatives thereof, and in particular teaches enzymatic methods of oxidizing 5-(hydroxymethyl)furfural using a galactose oxidase and/or peroxygenase. Even more specifically, WO 2014/015256 A2 discloses a method of oxidizing 5-(hydroxymethyl)furfural, comprising contacting 5-(hydroxymethyl)furfural with a galactose oxidase in a reaction mixture under suitable conditions to provide 2,5-diformylfuran.

Galactose oxidase catalyzes the oxidation of primary alcohols to the respective aldehydes paralleled by the reduction of O₂ to H₂O₂, see Paukner, R. et al. PLoS ONE, 2014, 9, e100116/1. Generally, 5-(hydroxymethyl)furfural can be oxidized in the presence of an oxidoreductase as biocatalyst. Galactose oxidase is a preferred oxidoreductase due to its fairly broad substrate range. As galactose oxidase genes have been already heterologously expressed in numerous hosts, galactose oxidase is generally available for synthetic purposes. The enzyme code for galactose oxidase according to International Union of Biochemistry and Molecular Biology is EC 1.1.3.9.

Preferred oxidoreductases used for the oxidation of 5-(hydroxymethyl)furfural to 2,5-diformylfuran are generally those acting on the CH-OH-group of the donor (EC 1.1), and preferably those using molecular oxygen as terminal electron acceptor (EC 1.1.3) In processes of preparing 2,5-diformylfuran from 5-(hydroxymethyl)furfural (HMF) there is typically produced an aqueous mixture comprising dissolved 5-(hydroxymethyl)furfural and dissolved 2,5-diformylfuran and a buffer substance. In such an aqueous mixture, typically present are also an oxidoreductase as biocatalyst for the oxidation of 5-(hydroxymethyl)furfural to 2,5-diformylfuran as well as in many cases horse radish peroxidase and catalase, which are commonly used as accessory enzyme or protective agents in order to prevent or alleviate oxidative stress and correspondingly assist in maintaining the activity of the oxidoreductase for an extended period of time. The 2,5-diformylfuran (DFF) present in such an aqueous reaction or product mixture in industrial or laboratory scale processes needs to be separated from the other constituents of the aqueous mixture in order to arrive at the isolated reaction product in solid form. Furthermore, the other constituents of the reaction or product mixture, in particular, any starting material (5-(hydroxymethyl)furfural) and any oxidoreductase, horse radish peroxidase and/or catalase should be separated from the reaction product 2,5-diformylfuran in order to provide a fraction of the aqueous mixture that is 2,5-diformylfuran depleted and therefore is ready for recycling into the oxidation process.

Correspondingly, it was a primary object of the present invention to provide a process of separating 2,5-diformylfuran from an aqueous mixture comprising dissolved 5-(hydroxymethyl)furfural, dissolved 2,5-diformylfuran and a buffer substance (and typically also one or more compounds selected from the group consisting of oxidoreductase, horse radish peroxidase and catalase).

According to a further aspect, it was an object of the present invention to provide for a specific process of biocatalytic oxidation of 5-(hydroxymethyl)furfural to 2,5-diformylfuran, wherein 2,5-diformylfuran is prepared in an aqueous mixture by oxidation of 5-(hydroxymethyl)furfural and is separated by a process according to the primary object of the present invention. Preferably, such a process of biocatalytic oxidation of 5-(hydroxymethyl)furfural to 2,5-diformylfuran should comprise a recycling step, wherein the aqueous mixture remaining after separation of 2,5-diformylfuran or a fraction thereof is fed back (i.e. recycled) into the oxidation reaction or at least provides the opportunity for recycling.

The primary object of the present invention as identified above is achieved by a process of separating 2,5-diformylfuran from an aqueous mixture comprising
- dissolved 5-(hydroxymethyl)furfural (HMF),
- dissolved 2,5-diformylfuran (DFF),
   and
- a buffer substance,
comprising the step of
(a) cooling down the mixture so that 2,5-diformylfuran precipitates.

Herein the aqueous mixture preferably additionally comprises one or more compounds selected from the group consisting of
- oxidoreductase (biocatalyst for oxidation of 5-(hydroxymethyl)furfural to 2,5-diformylfuran)
- horse radish peroxidase (accessory enzyme), and
- catalase (protecting agent for oxidoreductase).

The invention is based on the surprising discovery that 2,5-diformylfuran (DFF) can effectively be separated from an aqueous mixture of 5-(hydroxymethyl)furfural and 2,5-diformylfuran as typically produced during biocatalytic oxidation of 5-(hydroxymethyl)furfural by cooling down the mixture so that 2,5-diformylfuran selectively precipitates.

The temperature to which the mixture is cooled down in step (a) so that 2,5-diformylfuran precipitates depends on the individual practical situation. Preferably, the mixture is cooled down by using a cooling device having a cold surface. Preferably, and this is in accordance with the results of own experiments, the mixture is cooled down to a temperature below 20 °C, preferably below 15 °C, more preferably to a temperature of 10 °C. In specific situations it is even preferred to cool the mixture down to a temperature below 6°C, preferably to a temperature of 4 °C or lower. At such low temperatures precipitation occurs rapidly and selectively. In particular, in typical practical situations 5-(hydroxymethyl)furfural does not precipitate under these conditions. Preferably, the mixture is cooled down by at least 4K, preferably at least 6K, preferably by using a cooling device having a cold surface.

For preferred apparatus and equipment see below. Throughout the present text, the term "cooling down" means that the temperature of the mixture is decreased from a first (higher) temperature to a second (lower) temperature by means of cooling, preferably by using a cooling apparatus or device interacting with the vessel in which the aqueous mixture comprising 5-(hydroxymethyl)furfural and 2,5-diformylfuran is kept.

The cooling can be accomplished preferentially by exposing the mixture to a cold surface and/or by evaporative cooling achieved by lowering the pressure.

Further information regarding the compounds oxidoreductase, horse radish peroxidase and catalase, which preferably are present in the aqueous mixture, is provided below in the context of the related process of the present invention of biocatalytic oxidation.
Preferably, in a process of separating 2,5-diformylfuran from an aqueous mixture according to the present invention, the process comprises the additional step of
(b) separating 2,5-diformylfuran precipitated according to step (a) from the remaining aqueous mixture, to give separated precipitated 2,5-diformylfuran and a separated aqueous mixture comprising an amount of 2,5-diformylfuran not precipitated in step (a).

Depending on the circumstances of the individual situation, the separated aqueous mixture in addition to the amount of 2,5-diformylfuran not precipitated in step (a) comprises 5-(hydroxymethyl)furfural and a buffer substance as already present in the aqueous mixture before step (a). Furthermore, if already present before step (a) the separated aqueous mixture will also comprise oxidoreductase, horse radish peroxidase and catalase.

For separating 2,5-diformylfuran precipitated according to step (a) from the remaining aqueous mixture in step (b), the skilled person selects a separation technique matching with the demands of the individual situation, and typically the skilled person uses a separation technique belonging to the common general knowledge. Preferably, 2,5-diformylfuran precipitated according to step (a) is separated from the remaining aqueous mixture by means of filtration or centrifugation. Preferred is also a separation technique, wherein the remaining aqueous mixture or a fraction thereof is decanted or transfused or drained off from the precipitated 2,5-diformylfuran.

The concentration of 2,5-diformylfuran not precipitated in step (a) and therefore present in the remaining aqueous mixture separated in step (b) depends on the individual circumstances. In order to increase the total yield of 2,5-diformylfuran, it is typically preferred to also recover this 2,5-diformylfuran which is still dissolved in the remaining aqueous mixture, or a fraction thereof. Correspondingly, a process of the invention is preferred which comprises the additional step of
(c) evaporating water from an aqueous mixture comprising an amount of 2,5-diformylfuran not precipitated in step (a),
wherein this aqueous mixture of step (c) is
- the separated aqueous mixture as formed in step (b) or
- an aqueous mixture prepared from the separated aqueous mixture formed in step (b) by additional treatment steps.

By evaporating water from the aqueous mixture comprising an amount of 2,5-diformylfuran not precipitated in step (a), the concentration of 2,5-diformylfuran (DFF) in the mixture increases so that the saturation concentration is reached or exceeded. Then, additional amounts of 2,5-diformylfuran precipitate under the conditions given or, if applicable, precipitate upon cooling. Any precipitated and/or recovered 2,5-diformylfuran is preferably subjected to a purification routine. Such purification routine preferably comprises recrystallizing precipitated 2,5-diformylfuran and/or washing precipitated 2,5-diformylfuran with suitable washing liquids (aqueous solutions and/or organic organic solvents).

The process according to the present invention of separating 2,5-diformylfuran from an aqueous mixture is useful whenever an aqueous mixture comprising dissolved 5-(hydroxymethyl)furfural and dissolved 2,5-diformylfuran and a buffer substance are present. However, the process according to the present invention of separating 2,5-diformylfuran from an aqueous mixture has a particularly high value when it is used in the context of a process of biocatalytic oxidation of 5-(hydroxymethyl)furfural to 2,5-diformylfuran. This is in accordance with the further aspect of the present invention as discussed above.

Correspondingly, the further object of the present invention as identified above is achieved by a process of biocatalytic oxidation of 5-(hydroxymethyl)furfural to 2,5-diformylfuran, comprising the following steps:
(i) in a first vessel or first vessel unit oxidizing 5-(hydroxymethyl)furfural to 2,5-diformylfuran in an aqueous reaction mixture further comprising an oxidoreductase as biocatalyst and a buffer substance, by supplying oxygen to the reaction mixture so that an aqueous mixture is formed comprising
   - dissolved 5-(hydroxymethyl)furfural,
   - dissolved 2,5-diformylfuran
   - an oxidoreductase as biocatalyst (one or more oxidoreductase compounds, see below), and
   - a buffer substance,
(ii) separating 2,5-diformylfuran from the aqueous mixture formed in step (i) or from an aqueous mixture prepared by a process of the present invention as defined above of separating 2,5-diformylfuran from an aqueous mixture.

Insofar, the present invention relates to a process of biocatalytic oxidation of 5-(hydroxymethyl)furfural to 2,5-diformylfuran, wherein in step (i) an oxidoreductase is used as a biocatalyst. Oxidoreductase has the enzyme code EC 1 according to the conventions of the International Union of Biochemistry and Molecular Biology. Preferred oxidoreductases for use in the process of the present invention generally are those having enzyme code EC 1.1., i.e. are those acting on the CH-OH-group of the donor. More preferably, oxidoreductases with enzyme code EC 1.1.3 are used, i.e. those oxidoreductases using molecular oxygen as the terminal electron acceptor. Even more preferably, galactose oxidase having enzyme code EC 1.1.3.9 is used. Galactose oxidase is a copper dependent, monomeric enzyme. In nature, it is often found to be secreted by filamentous fungi. Galactose oxidase catalyses the oxidation of primary alcohols to the respective aldehydes paralleled by the reduction of O₂ to H₂O₂, see again Paukner, R. et al. PLoS ONE, 2014, 9, e100116/1. In document WO 2016/150629 it is disclosed how a galactose oxidase having enzyme code EC 1.1.3.9 can be prepared or provided. Furthermore, this document discloses a preferred enzyme of enzyme class 1.1.3.9. Biocatalysts of enzyme class EC 1.1.3.9 as disclosed in WO 2016/150629 are also preferred in the processes of the present invention.

Throughout the present text, when defining the invention or preferred embodiments thereof, the term "oxidoreductase" designates a single oxidoreductase compound or a mixture of two or more oxidoreductase compounds, if not stated otherwise.

As known from own experiments, a significant problem with currently used processes of biocatalytic oxidation of 5-(hydroxymethyl)furfural to 2,5-diformylfuran is that the biocatalyst used is prone to deterioration by both, the starting material 5-(hydroxymethyl)furfural (and/or detrimental by-products from 5-(hydroxymethyl)furfural synthesis) and the reaction product 2,5-diformylfuran, in particular if present in high concentrations at increased temperature. This means that a biocatalyst is used which under the reaction conditions loses its biocatalytic properties or is even completely destroyed. In view of the problems encountered as set forth above, and in accordance with the further aspect of the present invention as stated above, the present invention is based on the discovery that by separating 2,5-diformylfuran from an aqueous mixture comprising 2,5-diformylfuran and the biocatalyst, the biocatalyst is protected against the inhibitory potential of the 2,5-diformylfuran. Correspondingly, by separating the 2,5-diformylfuran one of the causes for deterioration and/or inhibition of the biocatalyst is at least alleviated. As a result of the separation of 2,5-diformylfuran, the biocatalyst is protected from deterioration or inhibition, so that is can be used for an extended period of time in a continuous, semi-continuous or discontinuous process.

The buffer substances present as a constituent of the aqueous mixture, which is subject to the separation process of the present invention, can be selected from a broad range of buffer substances known from the prior art as being acceptable in combination with biocatalysts.

The buffer substances can be exogenous or endogenous which means that they are either deliberately added (exogenous) in order to adjust specific parameters of the aqueous mixture and/or allow for specific reactions (e. g. for the conversion of 5-(hydroxymethyl)furfural to 2,5-diformylfuran) or are present in the aqueous mixture simply because they were added or produced in earlier steps of an overall procedure (e. g., humic acids as typically prepared during 5-(hydroxymethyl)furfural synthesis have a buffering effect and therefore typically constitute endogenous buffer substances). Preferred are aqueous mixtures comprising a sufficient amount of endogenous and/or exogenous buffer substances buffering at pH 7.0 so that by addition of 0,5 mmol HCl per liter the pH does not drop below pH 6.0 and by addition of 0,5 mmol NaOH per liter the pH does not rise above 8.0.

In particular, suitable buffer substances are those disclosed in WO 2014/015256 and/or are selected from the group consisting of alkali or alkaline earth metal hydrogencarbonate and/or carbonate, 1,4-piperazinediethanesulfonic acid (PIPES), 4-morpholinepropanesulfonic acid (MOPS), 4-(2-hydroxyethyl)-piperazineethane-sulfonic acid (HEPES), triethanolamine, tris(hydroxymethyl)aminomethane (TRIS), and phosphates. Ammonium, potassium, and sodium hydrogenphosphates are particularly preferred phosphate buffers for use as a constituent of the reaction mixture. By using a buffer substance, preferably potassium and/or sodium phosphate, the pH of the aqueous reaction mixture is preferably adjusted to and/or buffered in a pH range of from 5 to 9, more preferably 6 to 8, most preferably 6.5 to 7.5. Preferably, the total concentration of the preferred buffer substances potassium phosphate and sodium phosphate in the aqueous reaction mixture is in the range of from 10 to 200 mM, more preferably in the range of from 50 to 120 mM. In own experiments, a high degree of conversion was reached when potassium phosphate and/or sodium phosphates were used at 100 mM. Correspondingly, these buffer substances are particularly preferred.

As stated above, the aqueous mixture formed in step (i) comprises an oxidoreductase as biocatalyst. Correspondingly, the presence of one or more accessory enzymes like, e.g., horse radish peroxidase and/or catalase is also preferred in the aqueous mixture. Thus, a process of biocatalytic oxidation of the present invention is preferred, wherein the aqueous mixture formed in step (i) additionally comprises one or more accessory enzyme selected from the group consisting of horse radish peroxidase and catalase. Horse radish peroxidase (HRP) increases the catalytic performance of the main oxidoreductase. Catalase is an additive which reacts with and/or removes H₂O₂ produced during oxidation and therefore reduces oxidative stress.

A process of the invention of biocatalytic oxidation is preferred (preferably a process as defined above as being preferred), wherein the reaction temperature in oxidation step (i) is in the range of from 10 to 50°C, preferably in the range of from 15 to 40°C, more preferably in the range of from 20 to 30°C.

These reaction temperatures in own experiments allowed to arrive at a high yield of 2,5-diformylfuran, a high degree of conversion, and a high stability of the biocatalyst used.

A process of the present invention allows for a high degree of conversion of the starting material 5-(hydroxymethyl)furfural. Preferably, in a process of the present invention, the reaction is allowed to proceed in step (i) until at least 50 % of the total amount of 5-(hydroxymethyl)furfural employed is converted, preferably until at least 90 % of the total amount of 5-(hydroxymethyl)furfural employed is converted.

In order to reach a high degree of conversion and in order to protect the biocatalyst used, a process of the invention of biocatalytic oxidation is typically conducted at a specified pH-value. A process of the invention is preferred, wherein the pH in the aqueous reaction mixture present in the first vessel or first vessel unit is in the range of from 6 to 8, preferably 6.5 to 7.5
and/or (preferably "and")
wherein the buffer substance buffers in the range of from 6 to 8, preferably 6.5 to 7.5.

Preferred is a process of biocatalytic oxidation of the present invention, wherein the process is a continuous or semi-continuous process. Herein, preferably, 5-(hydroxymethyl)furfural (HMF) is continuously added to the first vessel or first vessel unit and/or water is continuously added to the first vessel or first vessel unit. Preferably, a mixture of 5-(hydroxymethyl)furfural and water is continuously added to the first vessel or first vessel unit employed in step (i).

A process of the present invention of biocatalytic oxidation is preferred, wherein
- material is drained from the aqueous mixture formed in step (i) in the first vessel or first vessel unit (e.g. a fraction of said aqueous mixture), and
- said step (ii) of separating 2,5-diformylfuran from the aqueous mixture formed in step (i) or from an aqueous mixture prepared or separated therefrom is conducted in a second vessel or second vessel unit.

Preferably, the material drained from the aqueous mixture formed in step (i) in the first vessel or first vessel unit is a fraction of the aqueous reaction mixture present in the first vessel or first vessel unit in step (i), comprising 2,5-diformylfuran and preferably (i) having the same composition as said aqueous mixture or (ii) comprising a reduced concentration of biocatalyst (e.g. the biocatalyst is prevented from draining by means of filtration).

A preferred process of biocatalytic oxidation as described is conducted in an apparatus comprising at least a first vessel or first vessel unit for step (i) and a second vessel or second vessel unit for step (ii). Preferably, the material drained from the aqueous mixture formed in step (i) is directly fed from the first vessel or first vessel unit into the second vessel or second vessel unit, preferably by means of a feed line connecting the vessels or vessel units, respectively. Optionally, the feed line comprises a filtration unit for removing biocatalyst from the material to be fed to the second vessel or vessel unit. In the first vessel or first vessel unit, the oxidation reaction (step (i)) is conducted in order to produce 2,5-diformylfuran (DFF), and in the second vessel or second vessel unit, step (ii) of separating 2,5-diformylfuran (DFF) is conducted.

Preferably, the aqueous mixture remaining after 2,5-diformylfuran in step (ii) is separated, is
- at least partially fed back to the first vessel or vessel unit (e.g. by means of a feed line)
   and/or
- subjected to further separation conditions so that further amounts of 2,5-diformylfuran are separated.

Regarding the preferred step of subjecting the aqueous mixture remaining after 2,5-diformylfuran in step (ii) is separated to further separation conditions, so that further amounts of 2,5-diformylfuran are separated, reference is made to the above description regarding the process of separating 2,5-diformylfuran from an aqueous mixture of the present invention, and in particular to the description regarding step (c). The above explanations apply also to the present preferred step, mutatis mutandis.

Preferably, the temperature in the first vessel or first vessel unit employed in step (i) is at least 4K higher than the temperature employed in the second vessel or second vessel unit employed in step (ii) in accordance with step (a) of the process according to the present invention of separating 2,5-diformylfuran from an aqueous mixture. Preferably, the temperature difference is at least 6K, and even more preferably, the temperature difference is at least 8K. By using a large temperature difference, the separation by precipitation of 2,5-diformylfuran occurs in a highly effective manner.

Preferably, in a process of the present invention in the second vessel or vessel unit a cold surface is provided having a temperature of at least 4K below the temperature in said first vessel or vessel unit, preferably at least 6K below the temperature in said first vessel or vessel unit.

It is preferred that the aqueous mixture remaining after in step (ii) 2,5-diformylfuran is separated (by conducting the process of the present invention of separating 2,5-diformylfuran from an aqueous mixture, i.e. in particular by cooling down the temperature of the mixture so that 2,5-diformylfuran precipitates in accordance with step (a) of such process) is
- at least partially fed back (i.e. recycled) to the first vessel or vessel unit
   and/or
- subjected to further separation conditions so that further amounts of 2,5-diformylfuran are separated, preferably followed by feeding back the further 2,5-diformylfuran depleted aqueous mixture to the first vessel or vessel unit.

The step of feeding back the aqueous mixture remaining after 2,5-diformylfuran in step (ii) is separated to the first vessel or vessel unit corresponds to recycling the aqueous mixture after removal of 2,5-diformylfuran. Correspondingly, it is preferred that simultaneously 5-(hydroxymethyl)furfural (HMF) is added to the first vessel or vessel unit (as described above), preferably in a continuous manner, in order to at least partly compensate for the consumption of 5-(hydroxymethyl)furfural by its conversion to 2,5-diformylfuran.

Particularly preferred is a process according to the present invention (as described above, preferably as described above as being preferred) of biocatalytic oxidation, comprising the following steps:
(i) in a first vessel or first vessel unit oxidizing 5-(hydroxymethyl)furfural (HMF) to 2,5-diformylfuran (DFF) in an aqueous reaction mixture further comprising an oxidoreductase as biocatalyst and a buffer substance, by supplying oxygen to the reaction mixture so that an aqueous mixture is formed comprising
   - dissolved 5-(hydroxymethyl)furfural (HMF),
   - dissolved 2,5-diformylfuran (DFF),
   - an oxidoreductase as biocatalyst (one or more oxidoreductase compounds, see above),
      and
   - a buffer substance (preferably as defined above; for further constituents which are preferably present see above)
(ii) separating 2,5-diformylfuran from the aqueous mixture formed in step (i) or from an aqueous mixture prepared or separated therefrom by a process according to the present invention of separating 2,5-diformylfuran from an aqueous mixture, as defined above and in the attached claims,
wherein the process of biocatalytic oxidation is a continuous or semi-continuous process, wherein
- material is continuously drained from the aqueous mixture formed in step (i) in the first vessel or first vessel unit,
wherein (preferably after directly feeding the material into a second vessel or vessel unit as described above)
- said step (ii) of separating 2,5-diformylfuran from the aqueous mixture formed in step (i) or from an aqueous mixture prepared or separated therefrom is continuously conducted in a second vessel or second vessel unit,
   and wherein
continuously at least a fraction or part of the aqueous mixture remaining after in step (ii) 2,5-diformylfuran is separated is fed back to the first vessel or vessel unit or is subjected to further separation conditions so that further amounts of 2,5-diformylfuran are separated.

A process according to the present invention of biocatalytic oxidation is preferred (preferably a process as defined above as being preferred), wherein the oxygen in the oxidation step (i)
is supplied to the first vessel or vessel unit as O₂ as a technically pure gas or in a gas mixture, preferably as O₂ in air
and/or (preferably "and")
is supplied to the first vessel or vessel unit in a continuous manner.

In many cases, it is preferred to bubble an O₂ containing gas mixture through the aqueous reaction mixture present in the first vessel or first vessel unit in step (i), preferably in a continuous manner.

Preferred is a process of the present invention of biocatalytic oxidation (as defined above, preferably as defined above as being preferred), wherein 5-(hydroxymethyl)furfural is continuously added to the first vessel or vessel unit.

Preferably, a process of the invention of biocatalytic oxidation of 5-(hydroxymethyl)furfural to 2,5-diformylfuran (preferably a process as defined above as being preferred) is conducted so that in step (i), i.e. in the first vessel or first vessel unit, no 2,5-diformylfuran precipitates under the selected reaction conditions. E.g., the saturation concentration of 2,5-diformylfuran in water at a temperature of 17 °C is approximately 10 g/L (see K.J. Zeitsch, The chemistry and technology of furfural and its many by-products, 2000, p. 256). Own experiments have shown that at a temperature of 30 °C the saturation concentration (maximum solubility) is approximately 15 g/L.

Correspondingly, and in particular when the concentration of 5-(hydroxymethyl)furfural in step (i), i.e. in the first vessel or first vessel unit is 10 g/L or higher, the process according to the present invention of biocatalytic oxidation by conducting step (ii) helps to avoid precipitation of 2,5-diformylfuran (DFF) from the aqueous reaction mixture present in the first vessel or first vessel unit during step (i).

As discussed above, step (ii) is preferably conducted in a second vessel or second vessel unit after material is drained from the aqueous mixture formed in step (i) in the first vessel or first vessel unit.
The present invention also relates to an apparatus for use in a process according to the present invention of biocatalytic oxidation, as defined in the claims and as described above (preferably as described above as being preferred). Such apparatus of the present invention comprises
(1) a first vessel or first vessel unit having
   - an oxygen inlet for supplying oxygen to the first vessel or vessel unit,
   - an outlet for draining material from the first vessel or vessel unit,
   - at least one inlet for feeding material to the first vessel or vessel unit,
(2) a second vessel or second vessel unit having
   - an inlet for feeding material to the second vessel or second vessel unit, this inlet being in fluid communication with said outlet for draining material from the first vessel or vessel unit, preferably via a first feed line,
   - an outlet for draining material from the second vessel or vessel unit, this outlet preferably being in fluid communication with an inlet for feeding material to the first vessel or vessel unit, preferably via a second feed line,
   - a device with a cold surface for adjusting the temperature within the second vessel,
(3) one or more control devices for controlling a continuous or semi-continuous operation of the oxidation process.

Herein, preferably, the apparatus of the present invention is ready for use or already in use, i.e. it preferably is an apparatus already comprising starting materials and optionally also reaction product. Thus, the preferred apparatus is an apparatus, wherein
the second vessel or second vessel unit is charged with an aqueous mixture comprising
- dissolved 5-(hydroxymethyl)furfural,
- dissolved 2,5-diformylfuran
- optionally an oxidoreductase as biocatalyst,
   and
- optionally a buffer substance,
and/or
the first vessel or first vessel unit is charged with 5-(hydroxymethyl)furfural in an aqueous reaction mixture further comprising an oxidoreductase as biocatalyst.

The present invention also relates to the use of an apparatus of the present invention (as defined in the claims and above, preferably as defined above as being preferred) in a process according to the present invention of biocatalytic oxidation as defined in the claims and above (preferably as defined above as being preferred).

Throughout the present text, features identified as being preferred in the context of a process according to the present invention of separating 2,5-diformylfuran from an aqueous mixture are also preferred with respect to the process according to the present invention of biocatalytic oxidation of 5-(hydroxymethyl)furfural to 2,5-diformylfuran, and vice versa. Likewise, features identified as being preferred in the context according to a process of the present invention are also preferred with respect to the apparatus and with respect to the use of the present invention, and vice versa. If not indicated otherwise, preferred features of a process of the present invention (process of separating 2,5-diformylfuran from an aqueous mixture or process of biocatalytic oxidation) are preferably combined with other preferred features of such a process.

Hereinafter, the invention is further described with respect to an example:

### Example: Process of biocatalytic oxidation of 5-(hydroxymethyl)furfural to 2,5-diformylfuran, including a process of separating 2,5-diformylfuran from an aqueous mixture:

### 1. Experimental Set-Up:

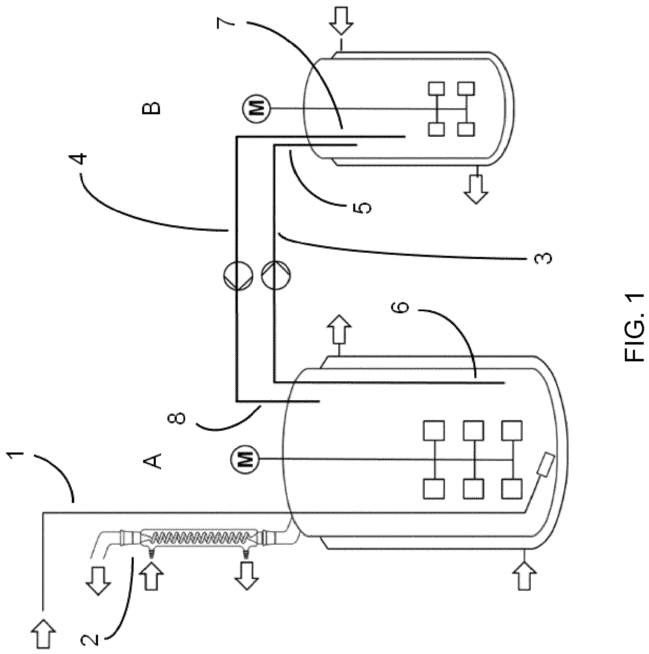

The experimental set-up is shown schematically in Figure 1.

In Figure 1, reference letter A designates a first jacketed glass vessel (first reactor) with a volume of 1 L, reference letter B desginates a second jacketed glass vessel (precipitation vessel as second reactor) having a volume of 0.25 L. Letters M designate mechanical stirrers for vessels A and B, respectively. Vessel A is connected to a gas inlet (1) for aeration of the vessel (oxygen inlet for supplying oxygen to the first vessel), and a corresponding cooled gas outlet (2). Vessels A and B are connected by means of a first connecting line (3) with a first pump for draining and transferring material from vessel A into vessel B, and by means of a second connecting line (4) with a second pump for draining and transferring material from vessel B to vessel A. Connecting lines (3) and (4) allow for continuous circulation of material between vessels A and B. Connecting line (3) comprises an inlet (5) for feeding material to the second jacketed glass vessel B (second vessel), this inlet being in fluid communication with an outlet (6) for draining material from the first jacketed glass vessel A (first vessel). Connecting line (4) comprises an outlet (7) for draining material from the second jacketed glass vessel B (second vessel), this outlet being in fluid communication with an inlet (8) for feeding material to the first jacketed glass vessel A (first vessel). By circulating a (heating or cooling) fluid through its jackets the temperature of the contents of the jacketed glass vessels A and B can be controlled. Optionally, the apparatus further comprises a control device for controlling a continuous or semi-continuous operation of the oxidation process (not shown in the figure).

### 2. Experimental Details:

### 2.1 Initial situation:

Vessel A was initially charged with the following materials to give a mixture:
800 ml 100 mM potassium phosphate buffer pH 7,
30.7 g (250 mM fin. conc.) HMF (5-(hydroxymethyl)furfural; > 80% purity; 5-(hydroxymethyl)furfural starting material was purified prior to use by dissolving in reaction buffer and mixing with 5 g activated carbon and 20 g poly-(1-phenylethylen) (tradename: crosspure F, by BASF) over night at 4°C; the mixture was filtered and used in the bioconversion)
1.107 µl (880 U/ml) catalase (Sigma) (protective agent; accessory enzyme)
7 mg (1.7 U/ml) peroxidase (Sigma) (accessory enzyme)
1.818 ml (0.05 mg/ml fin. conc.) galactose oxidase preparation (oxidoreductase biocatalyst).

Vessel B was initially charged with 200 ml 100 mM potassium phosphate buffer pH 7.

### 2.2 Oxidation of 5-(hydroxymethyl)furfural to 2,5-diformylfuran; precipitation of 2,5-diformylfuran:

The content of vessel A (initially, the mixture prepared according to 2.1) was continuously aerated with pressurized air (oxygen is present as O₂ in air) by means of the gas inlet (1), to cause oxidation, and stirred throughout the experiment (300 rpm). The content of vessel A (first vessel) was kept at a temperature of 20 °C, by using its jacket. The temperature of 20 °C was considered to be a favorable reaction temperature.

Throughout the experiment, the content of vessel B was kept at a temperature of 2 °C, by using its jacket, and stirred (50 rpm).

Mixed material was continuously circulated between vessels A and B at a rate of approximately 37 ml/min, using connecting lines (3) and (4). 2,5-diformylfuran was formed in vessel A (vessel) at 20 °C, in admixture with the other constituents of the reaction mixture present in vessel A. Aqueous reaction mixture comprising dissolved 5-(hydroxymethyl)furfural, dissolved 2,5-diformylfuran, galactose oxidase preparation as biocatalyst, and the buffer substance, was transferred to vessel B where the transferred mixture was cooled down to 2 °C in order to initiate precipitation. After a processing time of approximately six hours 2,5-diformylfuran had precipitated in vessel B (precipitation vessel). Throughout the experiment, an aqueous mixture comprising non-precipitated substances was continuously re-transferred (fed back) from vessel B to vessel A for further reaction.

### 2.3 Additional step: Evaporating water from aqueous mixture comprising an amount of 2,5-diformylfuran not precipitated; recovery of an additional amount of 2,5-diformylfuran:

A sample of the aqueous mixture comprising non-precipitated substances as present in vessel B was taken and water was evaporated therefrom by means of a rotavapor. Additional 2,5-diformylfuran precipitated in the form of an off-coloured material tainted with small amounts residual contaminants from the starting material.

### 3. Result:

The use of a cooled precipitation vessel (vessel B; first vessel; 2 °C) for separating 2,5-diformylfuran from an aqueous mixture comprising dissolved 5-(hydroxymethyl)furfural, dissolved 2,5-diformylfuran and a buffer substance favorably caused precipitation and, therefore, in situ-separation of 2,5-diformylfuran.

The corresponding continuous process of biocatalytic oxidation of 5-(hydroxymethyl)furfural to 2,5-diformylfuran gave 2,5-diformylfuran in a high yield, in particular if non-precipitated 2,5-diformylfuran as present in the aqueous mixture present in vessel B was precipitated by evaporating the water present in the mixture.

## Claims

1. Process of separating 2,5-diformylfuran from an aqueous mixture comprising
- dissolved 5-(hydroxymethyl)furfural,
- dissolved 2,5-diformylfuran
and
- a buffer substance,
comprising the step of
(a) cooling down the mixture so that 2,5-diformylfuran precipitates.

2. Process according to claim 1, wherein the mixture
- is cooled down to a temperature below 20 °C, preferably below 15 °C, more preferably to a temperature of 10 °C or lower
and/or
- is cooled down by at least 4K, preferably at least 6K.

3. Process according to any preceding claim, comprising the additional step of
(b) separating 2,5-diformylfuran precipitated according to step (a) from the remaining aqueous mixture, to give separated precipitated 2,5-diformylfuran and a separated aqueous mixture comprising an amount of 2,5-diformylfuran not precipitated in step (a).

4. Process according to claim 3,
comprising the additional step of
(c) evaporating water from an aqueous mixture comprising an amount of 2,5-diformylfuran not precipitated in step (a),
wherein this aqueous mixture of step (c) is
- the separated aqueous mixture as formed in step (b) or
- an aqueous mixture prepared from the separated aqueous mixture formed in step (b) by additional treatment steps.

5. Process of biocatalytic oxidation of 5-(hydroxymethyl)furfural to 2,5-diformylfuran, comprising the following steps:
(i) in a first vessel or first vessel unit oxidizing 5-(hydroxymethyl)furfural to 2,5-diformylfuran in an aqueous reaction mixture further comprising an oxidoreductase as biocatalyst and a buffer substance, by supplying oxygen to the reaction mixture so that an aqueous mixture is formed comprising
- dissolved 5-(hydroxymethyl)furfural,
- dissolved 2,5-diformylfuran
- an oxidoreductase as biocatalyst,
and
- a buffer substance,
(ii) separating 2,5-diformylfuran from the aqueous mixture formed in step (i) or from an aqueous mixture prepared or separated therefrom by a process as defined in any of the preceding claims.

6. Process according to claim 5, wherein the process is a continuous or semi-continuous process.

7. Process according to any of claims 5 to 6, wherein
- material is drained from the aqueous mixture formed in step (i) in the first vessel or first vessel unit, and
- said step (ii) of separating 2,5-diformylfuran from the aqueous mixture formed in step (i) or from an aqueous mixture prepared or separated therefrom is conducted in a second vessel or second vessel unit.

8. Process according to claim 7, wherein the aqueous mixture remaining after 2,5-diformylfuran in step (ii) is separated is
- at least partially fed back to the first vessel or vessel unit
and/or
- subjected to further separation conditions so that further amounts of 2,5-diformylfuran are separated.

9. Process according to any of claims 5 to 8,
wherein the temperature in said first vessel or first vessel unit used in step (i) is at least 4K, preferably at least 6K higher than the temperature reached in step (a)
and/or
wherein in the second vessel or vessel unit a cold surface is provided having a temperature of at least 4K below the temperature in said first vessel or vessel unit, preferably at least 6K below the temperature in said first vessel or vessel unit, or wherein cooling is accomplished by evaporative cooling achieved by lowering the pressure.

10. Process of biocatalytic oxidation of 5-(hydroxymethyl)furfural to 2,5-diformylfuran according to any of claims 5 to 9,
comprising the following steps:
(i) in a first vessel or first vessel unit oxidizing 5-(hydroxymethyl)furfural to 2,5-diformylfuran in an aqueous reaction mixture further comprising an oxidoreductase as biocatalyst and a buffer substance, by supplying oxygen to the reaction mixture so that an aqueous mixture is formed comprising
- dissolved 5-(hydroxymethyl)furfural,
- dissolved 2,5-diformylfuran
- an oxidoreductase as biocatalyst,
and
- a buffer substance,
(ii) separating 2,5-diformylfuran from the aqueous mixture formed in step (i) or from an aqueous mixture prepared or separated therefrom by a process as defined in any of claims 1 to 4,
wherein the process of biocatalytic oxidation is a continuous or semi-continuous process, wherein
- material is continuously drained from the aqueous mixture formed in step (i) in the first vessel or first vessel unit,
wherein
- said step (ii) of separating 2,5-diformylfuran from the aqueous mixture formed in step (i) or from an aqueous mixture prepared or separated therefrom is continuously conducted in a second vessel or second vessel unit,
and wherein
continuously the aqueous mixture remaining after in step (ii) 2,5-diformylfuran is separated is at least partially fed back to the first vessel or vessel unit and/or is subjected to further separation conditions so that further amounts of 2,5-diformylfuran are separated.

11. Process according to any of claims 5 to 10, wherein the oxygen in the oxidation step (i) is supplied to the first vessel or vessel unit as O₂ as a technically pure gas or in a gas mixture, preferably as O₂ in air
and/or
is supplied to the first vessel or vessel unit in a continuous manner.

12. Process according to any of claims 5 to 11, wherein 5-(hydroxymethyl)furfural is continuously added to the first vessel or vessel unit and/or water is continuously added to the first vessel or vessel unit.

13. Apparatus for use in a process according to any of claims 5 to 12, comprising
(1) a first vessel or first vessel unit having
- an oxygen inlet for supplying oxygen to the first vessel or vessel unit,
- an outlet for draining material from the first vessel or vessel unit,
- at least one inlet for feeding material to the first vessel or vessel unit,
(2) a second vessel or second vessel unit having
- an inlet for feeding material to the second vessel or second vessel unit, this inlet being in fluid communication with said outlet for draining material from the first vessel or vessel unit,
- an outlet for draining material from the second vessel or vessel unit,
- a device with a cold surface for adjusting the temperature within the second vessel,
(3) one or more control devices for controlling a continuous or semi-continuous operation of the oxidation process.

14. Apparatus according to claim 13, wherein
the second vessel or second vessel unit is charged with an aqueous mixture comprising
- dissolved 5-(hydroxymethyl)furfural,
- dissolved 2,5-diformylfuran
- an oxidoreductase as biocatalyst, and
- a buffer substance,
and/or
the first vessel or first vessel unit is charged with 5-(hydroxymethyl)furfural in an aqueous reaction mixture further comprising an oxidoreductase as biocatalyst.

15. Use of an apparatus according to any of claims 13 to 14 in a process according to any of claims 5 to 12.
